# EUROPEAN PATENT APPLICATION

(11) **EP 4 410 263 A1**
(43) Date of publication of application: **07.08.2024**
(21) Application number: 22875662.3
(22) Date of filing: 24.08.2022
(51) Int. Cl.: A61K 6/90

(54) **MAIN-MATERIAL PASTE AND DENTAL ALGINATE IMPRESSION MATERIAL**

(30) Priority: 30.09.2021 JP 2021162423
(71) Applicant: GC Corporation, Sunto-gun, Shizuoka 410-1307 (JP)
(72) Inventor: NIIZEKI, Naofumi, Tokyo, 174-8585 (JP); SHIBUYA, Yuki, Tokyo, 174-8585 (JP)
(74) Representative: Müller-Boré & Partner Patentanwälte PartG mbB
(86) International application number: PCT/JP2022/031832
(87) International publication number: WO 2023/053790

(57) **Abstract**

A base paste used in a dental alginate impression material contains an alginate, water, and a carboxylate containing 0 or more and 3 or less hydroxy groups and 3 or more and 8 or less carbon atoms.

## Description

### Technical Field

The present invention relates to a base paste used in a dental alginate impression material, and a dental alginate impression material.

### Background Art

In dental treatment, alginate impression materials are widely used as impression materials for taking the impression inside the mouth for producing a prosthesis. As the alginate impression materials, alginate impression materials containing: a base paste containing an alginate and water; and a curing-material paste containing a gelation reactant and a poorly water-soluble liquid compound are known (for example, see PTLs 1 and 2).

### Citation List

### Patent Literature

[PTL 1] International Publication No. WO 2012/063618
[PTL 2] International Publication No. WO 2014/050028

### Summary of Invention

### Technical Problem

However, existing alginate impression materials have problems in storage stability, such as deterioration of the base paste once the shelf life has passed, which may cause a time delay in initial setting when the materials are used.

An object of the present invention is to provide a base paste excellent in storage stability.

### Solution to Problem

A base paste according to an embodiment of the present invention is a base paste used in a dental alginate impression material, and contains an alginate, water, and a carboxylate containing 0 or more and 3 or less hydroxy groups and 3 or more and 8 or less carbon atoms.

### Advantageous Effects of Invention

According to an embodiment of the present invention, it is possible to provide a base paste excellent in storage stability.

### Description of Embodiments

An embodiment of the present invention will be described below in detail.

### [Base paste]

A base paste according to the present embodiment is used in a dental alginate impression material according to the present embodiment described below.

In the present specification, a dental alginate impression material is an impression-taking dental formulation utilizing a nature that when a water-soluble alginate and plaster are reacted, an insoluble calcium alginate is produced and cured. The base paste is a pasty base material forming a dental alginate impression material.

The base paste contains an alginate, water, and a carboxylate containing 0 or more and 3 or less hydroxy groups and 3 or more and 8 or less carbon atoms.

Examples of the alginate include: alkali metal salts of alginic acid, such as sodium alginate, potassium alginate, and the like; and ammonium salts of alginic acid, such as ammonium alginate, triethanol amine alginate, and the like. Among these alginates, alkali metal salts of alginic acid are preferable and potassium alginate is further preferable in terms of, for example, ease with procurement, ease with handling, and physical properties of a cured body of the dental alginate impression material according to the present embodiment described below.

Alginates may be used alone or in combination of two or more. Two or more may be used in combination.

The viscosity of a 1% by mass aqueous solution of the alginate at 20°C is preferably 0.005 Pa·s or higher and 0.5 Pa·s or lower and more preferably 0.1 Pa·s or higher and 0.4 Pa·s or lower. When the viscosity of the 1% by mass aqueous solution of the alginate at 20°C is 0.005 Pa·s or higher, a dental impression material using the base paste according to the present embodiment, when used in combination with an existing alginate impression material, can make impressions less likely to become broken.

When the viscosity of the 1% by mass aqueous solution of the alginate at 20°C is 0.005 Pa·s or higher and 0.5 Pa·s or lower, a mixed product of the base paste and a curing-material paste has a high fluidity, and the base paste according to the present embodiment, when used in combination with an alginate impression material, can improve the accuracy of impressions.

The content of the alginate in the base paste is not particularly limited, and is, for example, 1% by mass or greater and 20% by mass or less, preferably 3% by mass or greater and 18% by mass or less, and more preferably 5% by mass or greater and 15% by mass or less.

When the content of the alginate in the base paste is 1% by mass or greater, an alginate impression material (may hereinafter be referred to as dental impression material) using the base paste according to the present embodiment, when used in combination with an existing alginate impression material, can make impressions less likely to become broken. When the content of the alginate in the base paste is 20% by mass or less, the dental impression material using the base paste according to the present embodiment, when used in combination with an existing alginate impression material, can improve the accuracy of impressions.

As the water, for example, ion-exchanged water, distilled water, and the like may be used. Water sterilized with, for example, sodium hypochlorite or the like may also be used.

The content of the water in the base paste is not particularly limited, and is, for example, 75% by mass or greater and 99% by mass or less, preferably 80% by mass or greater and 97% by mass or less, and more preferably 85% by mass or greater and 95% by mass or less.

The carboxylate containing 0 or more and 3 or less hydroxy groups and 3 or more and 8 or less carbon atoms (hereinafter, may be referred to as the carboxylate) is a compound free of a hydroxyl group or containing from 1 through 3 hydroxyl groups, and containing at least one group resulting from hydrogen of a carboxy group being replaced with a monovalent alkali metal.

Examples of the monovalent alkali metal that forms the carboxylate include sodium, potassium, and the like.

Examples of the carboxylate include: saturated fatty acid salts such as propionate, butanoate, pentanoate, hexanoate, octanoate, and the like; unsaturated fatty acid salts such as 2-butenoate, 4-pentenoate, 5-hexenoate, 5-octenoate, 2,3-butadienoate, 2,4-pentadienoate, 2,4-hexadienoate (sorbate), 2,3-oactadienoate, 5,7-octadienoate, and the like; lactate; hydroxylates such as hydroxybutyrate, hydroxyvalerate, and the like; and aromatic carboxylates such as benzoate, hydroxybenzoate, phthalate, terephthalate, and the like.

The carbon chain that forms the carboxylate may be straight-chained or branched.

These carboxylates may be used alone or in combination of two or more.

Among these carboxylates, lactate, benzoate, and sorbate are preferable, and at least one selected from the group consisting of sodium lactate, potassium lactate, sodium benzoate, potassium benzoate, sodium sorbate, and potassium sorbate is more preferable.

The content of the carboxylate in the base paste is not particularly limited, and is, for example, 0.05% by mass or greater and 10% by mass or less, preferably 0.08% by mass or greater and 5% by mass or less, and more preferably 0.1% or greater and 2% by mass or less.

When the content of the carboxylate in the base paste is 0.05% by mass or greater, it is possible to improve the storage stability of the base paste, such that, for example, the base paste is less likely to deteriorate even after the shelf life has passed, and no time delay occurs in initial setting when the base paste is used. When the content of the carboxylate in the base paste is 10% by mass or less, it is possible to inhibit reduction in the viscosity of the base paste while restricting the viscosity to a low level.

The viscosity of the base paste at 20°C is preferably 6 Pa·s or higher and 60 Pa·s or lower and more preferably 8 Pa·s or higher and 55 Pa·s or lower. When the viscosity of the base paste is 6 Pa·s or higher, the accuracy of an impression is improved. When the viscosity of the base paste is 60 Pa·s or lower, the strength of a cured body of the dental impression material is improved.

The base paste according to the present embodiment has a lower viscosity than that of existing pasty alginate impression materials, and can be used in a combined impression method.

The base paste may further contain, for example, a curing retardant or the like. This makes the operable time of the dental impression material using the base paste according to the present embodiment long.

Examples of the curing retardant include phosphates such as trisodium phosphate, tripotassium phosphate, and the like, and condensed phosphates such as sodium pyrophosphate, potassium pyrophosphate, and the like. These curing retardants may be used alone or in combination of two or more.

The content of the curing retardant in the base paste is not particularly limited, and is, for example, 0.01% by mass or greater and 10% by mass or less, preferably 0.05% by mass or greater and 5% by mass or less, and more preferably 0.1% by mass or greater and 3% by mass or less.

When the content of the curing retardant in the base paste is 0.01% by mass or greater, the operable time of the dental impression material using the base paste according to the present embodiment is long. On the other hand, when the content of the curing retardant in the base paste is 10% by mass or less, the initial setting time of the dental impression material using the base paste according to the present embodiment is short, and the strength of a cured body of the dental impression material using the base paste according to the present embodiment is improved.

The base paste may contain other components as long as the object of the present invention is not missed. The base paste may further contain, for example, a bulking agent, a colorant, an antiseptic, a disinfectant, a fragrance, a pH adjuster, a surfactant, and the like as the other components.

Examples of the material that forms the bulking agent include clay minerals such as diatomaceous earth, talc, smectite, and the like, and inorganic oxides such as silica, alumina, and the like. These bulking agents may be used alone or in combination of two or more.

Examples of the surfactant include polyoxyethylene alkyl ethers, and the like. These surfactants may be used alone or in combination of two or more.

The consistency of the base paste is not particularly limited, yet is preferably 30 mm or greater. When the consistency of the base paste is 30 mm or greater, a mixed product of the base paste and a curing-material paste has a high fluidity. Hence, the dental impression material using the base paste according to the present embodiment, when used in combination with an existing alginate impression material, improves the accuracy of an impression.

As described above, the base paste according to the present embodiment contains an alginate, water, and a carboxylate containing 0 or more and 3 or less hydroxy groups and 3 or more and 8 or less carbon atoms. Hence, the base paste according to the present embodiment is less likely to deteriorate even after the shelf life has passed, and it is possible to obtain a base paste having a high storage stability such that, for example, no time delay occurs in the initial setting when the base paste is used.

The viscosity of the base paste according to the present embodiment can be restricted to be lower than that of existing pasty alginate impression materials, and it is possible to inhibit reduction in the viscosity while maintaining the viscosity at the low level. Hence, the base paste according to the present embodiment can be used in a combined impression method (i.e., a method of combining two uncured materials).

As described above, the base paste according to the present embodiment uses at least one selected from the group consisting of sodium lactate, potassium lactate, sodium benzoate, potassium benzoate, sodium sorbate, and potassium sorbate as the carboxylate. Hence, the base paste is even less likely to deteriorate after the shelf life has passed and can inhibit time delay in initial setting, and a base paste excellent in storage stability can be obtained.

### [Dental alginate impression material]

A dental alginate impression material according to the present embodiment contains a base paste and a curing-material paste.

As the base paste, the base paste according to the present embodiment described above is used. Specifically, the base paste contained in the dental alginate impression material according to the present embodiment contains an alginate, water, and a carboxylate containing 0 or more and 3 or less hydroxy groups and 3 or more and 8 or less carbon atoms.

The content of the alginate in the dental alginate impression material is not particularly limited, and is, for example, 0.5% by mass or greater and 15% by mass or less, preferably 2% by mass or greater and 10% by mass or less, and yet more preferably 3% by mass or greater and 8% by mass or less.

When the content of the alginate in the dental alginate impression material according to the present embodiment is 0.5% by mass or greater, the dental alginate impression material according to the present embodiment, when used in combination with an existing alginate impression material, makes an impression less likely to become broken. On the other hand, when the content of the alginate in the dental alginate impression material according to the present embodiment is 15% by mass or less, the dental alginate impression material according to the present embodiment, when used in combination with an existing alginate impression material, improves the accuracy of an impression.

The content of water in the dental alginate impression material according to the present embodiment is not particularly limited, and is, for example, 40% by mass or greater and 75% by mass or less, preferably 45% by mass or greater and 70% by mass or less, and more preferably 50% by mass or greater and 65% by mass or less.

The content of the carboxylate in the dental alginate impression material according to the present embodiment is not particularly limited, and is, for example, 0.01% by mass or greater and 7% by mass or less, preferably 0.03% by mass or greater and 5% by mass or less, and more preferably 0.05% by mass or greater and 1.5% by mass or less.

When the content of the carboxylate in the base paste is 0.01% by mass or greater, it is possible to improve the storage stability of the base paste, such that, for example, the base paste is less likely to deteriorate even after the shelf life has passed, and no time delay occurs in initial setting when the base paste is used. When the content of the carboxylate in the base paste is 7% by mass or less, it is possible to inhibit reduction in the viscosity of the base paste while restricting the viscosity to a low level.

### [Curing-material paste]

The curing-material paste is a material to be mixed with the base paste such that the dental alginate impression material is cured.

The curing-material paste contains a gelation reactant and a poorly water-soluble liquid compound

As the gelation reactant, a divalent or greater metal compound may be used.

The divalent or greater metal compound is not particularly limited, and examples of the divalent or greater metal compound include divalent or greater metals, salts, oxides, and hydroxides, and the like thereof, and the like. These divalent or greater metal compounds may be used alone or in combination of two or more.

Examples of the divalent or greater metals include calcium, magnesium, zinc, aluminum, iron, titanium, zirconium, tin, and the like.

Examples of the salts of the divalent or greater metals include calcium sulfate dihydrate (CaSO₄•2H₂O), calcium sulfate hemihydrate (CaSO₄• 1/2H₂O), anhydrous calcium sulfate anhydride (CaSO₄), and the like.

Examples of the oxides of the divalent or greater metals include calcium oxide, magnesium oxide, zinc oxide, titanium oxide, zirconium oxide, tin oxide, and the like.

Example of the hydroxides of the divalent or greater metals include calcium hydroxide, magnesium hydroxide, zinc hydroxide, aluminum hydroxide, iron hydroxide, and the like.

The content of the gelation reactant in the curing-material paste is not particularly limited, yet is preferably 10% by mass or greater and 60% by mass or less. When the content of the gelation reactant in the curing-material paste is 10% by mass or greater, the strength of a cured body of the dental alginate impression material using the curing-material paste is improved. When the content of the gelation reactant in the curing-material paste is 60% by mass or less, the operable time of the dental impression material is long.

The poorly-water-soluble liquid compound is not particularly limited as long as it can make the gelation reactant pasty. Examples of the poorly-water-soluble liquid compound include hydrocarbon compounds, aliphatic alcohols, aromatic alcohols, fatty acids, fatty acid esters, silicone oils, and the like. These poorly-water-soluble liquid compounds may be used alone or in combination of two or more.

In the present specification, poor water-solubility means a solubility of 5 g or less in water of 100 g at 20°C.

The hydrocarbon compound may be any of chained compounds and cyclic compounds. Examples of the hydrocarbon compound include hexane, heptane, octane, nonane, decane, undecane, dodecane, tridecane, tetradecane, pentadecane, 2,7-dimethyl octane, 1-octene, cycloheptane, cyclononane, kerosene, liquid paraffin, and the like.

The aliphatic alcohol may be any of saturated aliphatic alcohols and unsaturated aliphatic alcohols. Examples of the aliphatic alcohol include 1-hexanol, 1-octanol, citronellol, oleyl alcohol, and the like.

Examples of the aromatic alcohol include benzyl alcohol, m-cresol, and the like.

The fatty acid may be any of saturated fatty acids and unsaturated fatty acids.

Examples of the fatty acid include hexanoic acid, octanoic acid, oleic acid, linoleic acid, and the like.

The fatty acid ester may be any of saturated fatty acid esters and unsaturated fatty acid esters.

Examples of the fatty acid ester include ethyl octanoate, butyl phthalate, oleic acid glyceride, olive oil, sesame oil, liver oil, whale oil, and the like.

Examples of the silicone oil include polydimethyl siloxane, polymethyl phenyl siloxane, polymethyl hydrogen siloxane, polyphenyl hydrogen siloxane, and the like.

The content of the poorly-water-soluble liquid compound in the curing-material paste is preferably 10% by mass or greater and 50% by mass or less and yet more preferably 15% by mass or greater and 30% by mass or less. When the content of the poorly-water-soluble liquid compound in the curing-material paste is 10% by mass or greater, the operability of the dental alginate impression material according to the present embodiment using the curing-material paste is improved. When the content of the poorly-water-soluble liquid compound in the curing-material paste is 50% by mass or less, the strength of a cured body of the dental alginate impression material is improved.

It is preferable that the curing-material paste further contains polybutene. This improves the curability of the dental alginate impression material according to the present embodiment and makes the operable time of the dental alginate impression material long.

It is preferable that the polybutene is liquid. It is possible to synthesize the polybutene by copolymerizing isobutene and 1-butene. The number average molecular weight of the polybutene is preferably from 300 through 4,000.

The content of the polybutene in the curing-material paste is preferably 0.5% by mass or greater and 30% by mass or less and more preferably 1% by mass or greater and 20% by mass or less. When the content of the polybutene in the curing-material paste is 0.5% by mass or greater and 30% by mass or less, the operability of the dental alginate impression material according to the present embodiment is improved.

It is preferable that the curing-material paste further contains a curing retardant. This makes the operable time of the dental alginate impression material according to the present embodiment long.

Examples of the curing retardant include phosphates such as trisodium phosphate, tripotassium phosphate, and the like, and condensed phosphates such as sodium pyrophosphate, potassium pyrophosphate, and the like. These curing retardants may be used alone or in combination of two or more.

The content of the curing retardant in the curing-material paste is preferably 0.01% by mass or greater and 10% by mass or less. When the content of the curing retardant in the curing-material paste is 0.01% by mass or greater, the operable time of the dental alginate impression material according to the present embodiment is long. When the content of the curing retardant in the curing-material paste is 10% by mass or less, the initial setting time of the dental alginate impression material is short, and the strength of a cured body of the dental alginate impression material is improved.

The curing-material paste may further contain, for example, a bulking agent, a curing modifier, a surfactant, a colorant, an antiseptic, a disinfectant, a fragrance, a pH adjuster, and the like.

Examples of the material that forms the bulking agent include clay minerals such as diatomaceous earth, talc, smectite, and the like, and inorganic oxides such as silica, alumina, and the like. These bulking agents may be used alone or in combination of two or more.

Examples of the curing modifier include titanium potassium fluoride, potassium silicofluoride, and the like. These curing modifiers may be used alone or in combination of two or more.

Examples of the surfactant include polyoxyethylene alkyl ethers and the like. These surfactants may be used alone or in combination of two or more.

The consistency of the curing-material paste is not particularly limited, yet is preferably 30 mm or greater. When the consistency of the curing-material paste is 30 mm or greater, a mixed product of the base paste and the curing-material paste has a high fluidity. Hence, the dental alginate impression material according to the present embodiment, when used in combination with an existing alginate impression material, improves the accuracy of an impression.

### [Method of using dental impression material according to the present embodiment]

As a dental impression material according to the present embodiment, the base paste and the curing-material paste described above are used while being mixed.

For example, a mixed product obtained by the base paste and the curing-material paste, which are filled in separate packing containers, being extruded by a manual or electric extruder and passed through a nozzle, is applied to teeth.

Examples of the packing container include a resin or metal molding, a resin or metal bag, and the like.

The mass ratio of the base paste to the curing-material paste when mixing the base paste and the curing-material paste is not particularly limited, and is, for example, from 1 through 5, preferably from 1 through 3, and more preferably from 1 through 2.

The dental alginate impression material according to the present embodiment can obtain the effect of the base paste according to the present embodiment by containing the base paste according to the present embodiment and the curing-material paste as described above. That is, because the base paste contained in the dental alginate impression material according to the present embodiment has a high storage stability (i.e., less likeliness to deteriorate even after the shelf life has elapsed), it is possible to inhibit a time delay in initial setting of the dental alginate impression material when the base paste is used. Hence, the dental alginate impression material according to the present embodiment makes an impression less likely to become broken and improves the accuracy of the impression.

The base paste contained in the dental alginate impression material according to the present embodiment has a lower viscosity than that of existing pasty alginate impression materials, and reduction in the viscosity is inhibited while the viscosity is maintained at the low level. Hence, the dental alginate impression material according to the present embodiment can be used in a combined impression method.

### [For-combination impression material]

As a for-combination impression material according to the present embodiment, the dental alginate impression material according to the present embodiment described above can be used. In the present specification, a for-combination impression material is a dental formulation for taking an impression by a combined impression method (i.e., a method of combining two uncured materials).

The for-combination impression material according to the present embodiment is used in combination with any other alginate impression material. Specifically, the for-combination impression material according to the present embodiment is applied to an impression-taking target such as teeth and the like after the any other alginate impression material is applied to the target.

The any other alginate impression material is not particularly limited. For example, an alginate impression material used in an agar/alginate combined impression method may be used.

The any other alginate impression material may be a powder type, or may be a paste type containing: a base paste containing an alginate and water; and a curing-material paste containing a gelation reactant and a poorly-water-soluble liquid compound.

The mass ratio between the for-combination impression material according to the present embodiment and the any other alginate impression material is not particularly limited.

When a paste-type alginate impression material is used as the any other alginate impression material, this alginate impression material is the same as the dental alginate impression material according to the present embodiment except that the consistency of a mixed product of the base paste and the curing-material paste is less than 36 mm.

By the dental alginate impression material according to the present embodiment being used as the for-combination impression material according to the present embodiment as described above, the effect of the dental alginate impression material according to the present embodiment can be obtained. That is, because the base paste contained in the for-combination impression material according to the present embodiment has a high storage stability (i.e., less likeliness to deteriorate even after the shelf life has elapsed), it is possible to inhibit a time delay in initial setting of the dental alginate impression material when the base paste is used. Hence, an impression to be obtained is less likely to become broken.

The base paste contained in the dental alginate impression material as the for-combination impression material according to the present embodiment has a lower viscosity than that of existing pasty alginate impression materials, and reduction in the viscosity is inhibited while the viscosity is maintained at the low level. Hence, the for-combination impression material according to the present embodiment can improve the accuracy of an impression obtained by employing a combined impression method.

### Examples

The present invention will further be described below by way of Examples. In the following description, any numeral without a unit, and "part" or "%" are on a mass basis unless otherwise particularly specified. Evaluations of Examples and Comparative Examples were performed according to the tests described below.

### <Preparation of curing-material paste>

Calcium sulfate (45 parts), liquid paraffin (23 parts), silica (12.5 parts), magnesium hydroxide (6 parts), polybutene (4 parts), titanium potassium fluoride (3 parts), polyoxyethylene alkyl ether (3 parts), zinc oxide (2 parts), and tripotassium phosphate (1.5 parts) were mixed, to obtain a curing-material paste. The composition of the curing-material paste is indicated in Table 1.

**[Table 1]**

| Curing-material paste | % |
|---|---|
| Calcium sulfate | 45 |
| Liquid paraffin | 23 |
| Silica | 12.5 |
| Magnesium hydroxide | 6 |
| Polybutene | 4 |
| Titanium potassium fluoride | 3 |
| Polyoxyethylene alkyl ether | 3 |
| Zinc oxide | 2 |
| Tripotassium phosphate | 1.5 |

### <Preparation of base paste>

Potassium alginate (8 parts), a carboxylate containing 0 or more and 3 or less hydroxy groups and 3 or more and 8 or less carbon atoms, and potassium pyrophosphate serving as a curing retardant (0.2 parts) were mixed, and distilled water was added thereto such that the total would be 100 parts, to obtain a base paste. The viscosity at 20°C of a 1% by mass aqueous solution of the potassium alginate used was 144 mPa·s.

### <Storage property (viscosity)>

Using an E-type viscometer (obtained from Toki Sangyo Co., Ltd., RE-85) at a cone angle of 3 degrees at a shear rate of 5 (1/sec) (at a rotation rate of 10 rpm) at a temperature of 20°C, the viscosity of the base paste was measured before the base paste was stored and after it was stored in an environment at 60°C at a relative humidity of 100% for 1 week, to test the storage stability of the base paste. The storage stability was evaluated according to the criteria described below.

### [Evaluation criteria]

Good: 10 Pa·s or higher and lower than 50 Pa·s
Fail: Lower than 10 Pa·s or 50 Pa·s or higher

### <Initial setting time>

Before the base paste was stored and after it was stored in an environment at 60°C at a relative humidity of 100% for 1 week, the base paste and the curing-material paste were mixed at a mass ratio of 2 : 1, and the initial setting time of a dental alginate impression material was measured in compliance with "7.2 Initial setting time of Dental alginate impression material" stipulated by JIS T 6505. The initial setting time was evaluated according to the criteria described below.

### [Evaluation criteria]

Good: 50 s or longer and 120 s or shorter
Fail: Shorter than 50 s or longer than 120 s

### <Compressive strength>

Before the base paste was stored and after it was stored in an environment at 60°C at a relative humidity of 100% for 1 week, the base paste and the curing-material paste were mixed at a mass ratio of 2 : 1, and a cured body was produced in a thermostatic water tank at 35°C. Using an autograph (obtained from Shimadzu Corporation, EZ-20), the cured body was measured at a crosshead speed of 100 ± 20 N/min until the cured body became broken, to evaluate the compressive strength. The compressive strength was evaluated according to the criteria described below.

### [Evaluation criteria]

Excellent: 0.50 MPa or higher
Good: 0.35 MPa or higher and lower than 0.50 MPa
Fail: Lower than 0.35 MPa

Examples and Comparative Examples will be described below.

### [Example 1]

A base paste in which sodium benzoate (1 part) was blended as a carboxylate was prepared, and the storage property (viscosity), the initial setting time, and the compressive strength of the base paste were evaluated. The composition and the evaluation results of Example 1 are indicated in Table 2.

### [Example 2]

A base paste was prepared in the same manner as in Example 1 except that the blending amount of sodium benzoate was changed to 0.1 parts, and was evaluated. The composition and the evaluation results of Example 2 are indicated in Table 2.

### [Example 3]

A base paste was prepared in the same manner as in Example 1 except that sodium lactate (1 part) was blended instead of sodium benzoate, and was evaluated. The composition and the evaluation results of Example 3 are indicated in Table 2.

### [Example 4]

A base paste was prepared in the same manner as in Example 1 except that potassium sorbate (0.3 parts) was blended instead of sodium benzoate, and was evaluated. The composition and the evaluation results of Example 4 are indicated in Table 2.

### [Example 5]

A base paste was prepared in the same manner as in Example 1 except that sodium lactate (1 part) was further added, and was evaluated. The composition and the evaluation results of Example 5 are indicated in Table 2.

### [Comparative Example 1]

A base paste was prepared in the same manner as in Example 1 except no carboxylate was blended, and was evaluated. The composition and the evaluation results of Comparative Example 1 are indicated in Table 2.

**[Table 2]**

| Base paste | Ex. | | | | | Comp. Ex. |
|---|---|---|---|---|---|---|
| | 1 | 2 | 3 | 4 | 5 | 1 |
| Potassium alginate | 8 | 8 | 8 | 8 | 8 | 8 |
| Sodium benzoate | 1 | 0.1 | | | 1 | |
| Sodium lactate | | | 1 | | 1 | |
| Potassium sorbate | | | | 0.3 | | |
| Potassium pyrophosphate | 0.2 | 0.2 | 0.2 | 0.2 | 0.2 | 0.2 |
| Distilled water | 90.8 | 91.7 | 90.8 | 91.5 | 89.8 | 91.8 |
| Total | 100 | 100 | 100 | 100 | 100 | 100 |
| Storage property (viscosity) (Pa·s; 0 weeks) | 37 | 37 | 37 | 37 | 37 | 37 |
| | Good | Good | Good | Good | Good | Good |
| Storage property (viscosity) (Pa·s; 60°C, 1 week) | 13 | 13 | 13 | 12 | 12 | 8 |
| | Good | Good | Good | Good | Good | Fail |
| Initial setting time (sec.; 0 weeks) | 65 | 65 | 70 | 60 | 65 | 80 |
| | Good | Good | Good | Good | Good | Good |
| Initial setting time (sec.; 60°C, 1 week) | 70 | 70 | 80 | 70 | 70 | 150 |
| | Good | Good | Good | Good | Good | Fail |
| Compressive strength (Mpa; 0 weeks) | 0.72 | 0.71 | 0.67 | 0.64 | 0.70 | 0.47 |
| | EXC. | EXC. | EXC. | EXC. | EXC. | Good |
| Compressive strength (Mpa; 60°C, 1 week) | 0.54 | 0.52 | 0.53 | 0.44 | 0.49 | 0.28 |
| | EXC. | EXC. | EXC. | Good | Good | Fail |

From Table 2, the dental alginate impression materials using base pastes each containing an alginate, water, and a carboxylate containing 0 or more and 3 or less hydroxy groups and 3 or more and 8 or less carbon atoms achieved good results in all of storage stability, initial setting time, and compressive strength (Examples 1 to 5).

As compared with this, the dental alginate impression material using a base paste free of a carboxylate achieved fail results in all of storage stability, initial setting time, and compressive strength after storage at 60°C for 1 week (Comparative Example 1).

Embodiments of the present invention have been described above. However, the present invention is not limited to specific embodiments, and various modifications and changes are applicable within the scope of the invention described in the claims.

The present application claims priority to Japanese Patent Application No. 2021-162423 filed September 30, 2021, the entire contents of which are incorporated herein by reference.

## Claims

1. A base paste used in a dental alginate impression material, the base paste comprising:
an alginate;
water; and
a carboxylate containing 0 or more and 3 or less hydroxy groups and 3 or more and 8 or less carbon atoms.

2. The base paste according to claim 1,
wherein the carboxylate is at least one selected from the group consisting of sodium lactate, potassium lactate, sodium benzoate, potassium benzoate, sodium sorbate, and potassium sorbate.

3. A dental alginate impression material, comprising:
the base paste of claim 1 or 2; and
a curing-material paste containing a gelation reactant and a poorly-water-soluble liquid compound.
